# EUROPÄISCHE PATENTSCHRIFT

(11) **EP 0 697 208 B1**
(45) Veröffentlichungstag und Bekanntmachung des Hinweises auf die Patenterteilung: **09.10.1996**
(21) Anmeldenummer: 95111118.6
(22) Anmeldetag: 15.07.1995
(51) Int. Cl.: A61K 7/06

(54) **Mittel zur Haarbehandlung**
Hair treating composition
Composition pour le traitement des cheveux

(30) Priorität: 28.07.1994 DE 4426794
(43) Veröffentlichungstag der Anmeldung: 21.02.1996
(73) Patentinhaber: GOLDWELL GmbH, D-64297 Darmstadt (DE)
(72) Erfinder: Bünning, Einhard, D-64342 Seeheim-Jugenheim (DE)

(56) Entgegenhaltungen:
- EP-A- 0 524 612
- EP-A- 0 526 279

## Beschreibung

Die vorliegende Erfindung betrifft ein Mittel zur Haarbehandlung insbesondere zur Behandlung von gespaltenen Haarspitzen, mit verbesserten Gebrauchseigenschaften.

Gespaltene Haarspitzen sind eine häufig auftretende Erscheinungsform geschädigten Haares, insbesondere nach starker Beanspruchung durch Dauerwellen, Sonneneinstrahlung oder sonstige Wärmebehandlung oder auch mechanische und Umwelt-Einflüsse.

Zur Behandlung gespaltener Haarenden oder gerissenen Haares wurden bereits verschiedene Präparate vorgeschlagen, insbesondere auf Basis höherviskoser Öle, wodurch zwar ein gewisser Klebeeffekt, aber auch ein unschönes, fettiges Aussehen des so behandelten Haares bewirkt wird.

Aus der EP-A 285 364 sind Mittel zur Behandlung gerissener Haare bzw. von Haaren mit gespaltenen Spitzen bekannt, die aus einem hochmolekularen Silikon, vorzugsweise gelöst in einem Öl oder niedrigsiedenden Silikon, bestehen. Diese Zusammensetzungen sind ebenfalls bis zu einem gewissen Grad geeignet, gerissenes Haar oder gespaltene Haarspitzen zusammenzufügen, weisen jedoch aufgrund ihrer starken Hydrophie ebenfalls den Nachteil auf, nach der Behandlung nicht aus dem Haar auswaschbar zu sein. Dadurch verleihen sie dem Haar nicht nur ein unästhetisches Aussehen, sondern beeinträchtigen auch etwaige nachfolgende Haarbehandlungsverfahren, insbesondere Dauerwellung und Haarfäarbung.

Es bestand daher ein Bedürfnis nach Haarbehandlungsmitteln, die diese Nachteile nicht aufweisen.

Die vorliegende Erfindung löst diese Aufgabe durch ein Mittel zur Haarbehandlung, das nicht nur gespaltene Haarspitzen wieder schließt, sondern auch aus dem Haar gut auswaschbar ist und eine Lösung mindestens eines quaternierten Aminoalkyl-, insbesondere Aminopropyldimethylpolysiloxan/Polyethyloxazolin-Copolymerisats der Formel 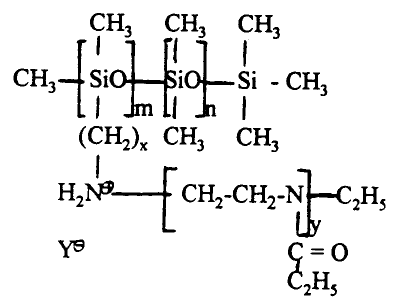 worin m und n jeweils ganze Zahlen von 20 bis 10 000, insbesondere 50 bis 7000, vor allem 100 bis 5000, x eine Zahl zwischen 1 und 5, vorzugsweise 3, und y eine Zahl von 5 bis 30 bedeuten, und Y⁻ ein Anion darstellt, in mindestens einem organischen Lösungsmittel enthält.

Bevorzugte organische Lösungsmittel sind in diesem Zusammenhang niedere Alkohole, d.h. Ethanol, n-Propanol und Isopropylalkohol. Die Konzentration des Pfropfcopolymeren in der Lösung liegt dabei vorzugsweise zwischen etwa 10 bis etwa 50, insbesondere etwa 20 bis etwa 40 Gew.-%, bezogen aufdie Gesamtzusammensetzung.

Die Herstellung der erfindungsgemäß zum Einsatz gelangenden Aminoalkyldimethylpolysiloxan/Polyethyloxazolin-Pfropfcopolymerisate erfolgt entsprechend dem folgenden Schema:

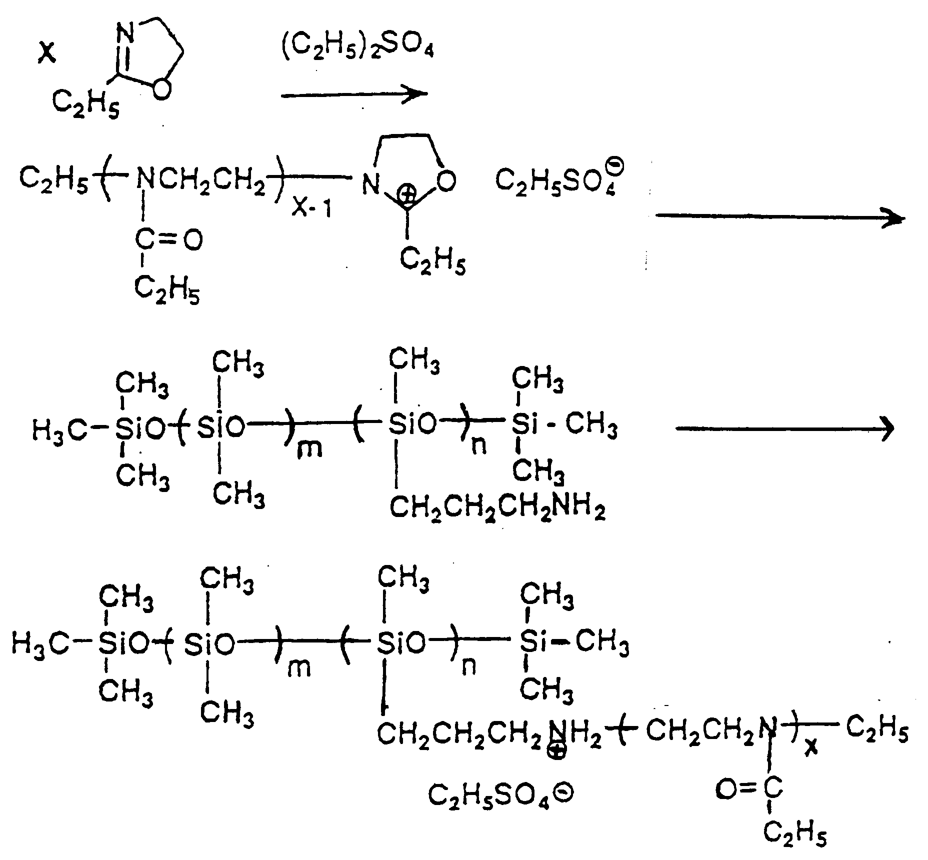

Das Anion Y⁻der allgemeinen Formel kann selbstverständlich auch ein anderes als das Ethylsulfat-Anion des obengenannten Beispiels sein, d.h. die Quaternierung kann auch mit Methylchlorid, Dimethylsulfat, Benzylchlorid, Dodecylbromid etc. erfolgen.

Ein besonders bevorzugtes Pfropfcopolymerisat der dargestellten Art weist ein Gesamtmolekulargewicht von etwa 50 000 bis etwa 150 000, vorzugsweise etwa 80 000 bis etwa 120.000, insbesondere etwa 100 000 Daltons auf, wobei das Molgewicht des Oxazolin-Segments etwa 2 500 bis etwa 7 500, vorzugsweise etwa 4 000 bis etwa 6 000, insbesondere etwa 5 000 Daltons/Segment beträgt, d.h. der Molanteil bei 20 Einheiten/Molekül liegt. Der bevorzugte Si-Gehalt beträgt, entsprechend der Elementaranalyse, etwa 50 %.

Die erfindungsgemäßen Zusammensetzungen enthalten vorzugsweise noch mindestens einen Kohlenwasserstoff mit einem Siedepunkt zwischen etwa 35 und etwa 280°C (bei Normaldruck) und/oder mindestens ein flüchtiges Silikon. Flüchtige Silikone sind insbesondere Dimethicone, Phenyl Dimethicone und Cyclomethicone der allgemeinen Formeln 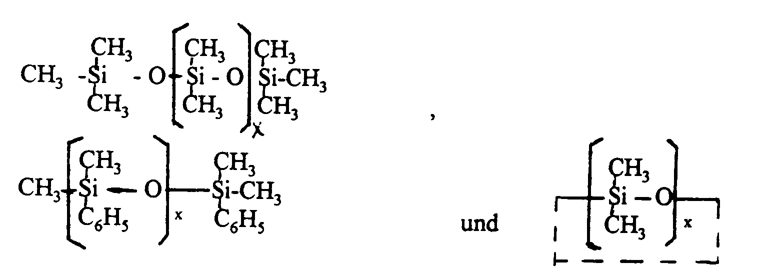 wobei x niedere Zahlen von etwa 1 bis etwa 10, im Falle des erstgenannten Dimethicones auch 0 bis 10 bedeuten.

Solche Produkte sind beispielsweise unter den Bezeichnungen "Dow Corning ^{R} 200, 225, 244, 235, 344, 345 Fluid" oder "SF-1202 oder SF-1204 Silicone Fluid" im Handel.

Die Viskositäten dieser flüchtigen Silikone liegen zwischen etwa 0,5 und etwa 500 cSt bei 25°C, insbesondere etwa 1 und etwa 100, vorzugsweise etwa 5 bis etwa 50 cSt bei 25°C.

Geeignete Kohlenwasserstofe sind Hexan, Isohexan, Heptan, Octan, Nonan, Decan, Dodecan, Tetradecan, Hexadecan, Octadecan und deren Isomere sowie Kohlenwasserstoff-Gemische, besonders bevorzugt ist Isohexadecan.

Der Anteil der flüchtigen Silikone bzw. Kohlenwasserstoffe im definierten Siedebreich liegt bei etwa 20 bis 60 Gew.-%, vorzugsweise 30 bis 50 Gew.-% der Gesamtzusammensetzung des Mittels.

Das erfindungsgemäße Mittel liegt als organische Lösung vor, die, falls erwünscht, natürlich auch auf an sich bekannte Weise als Aerosolspray konfektioniert sein kann.

Ebenso können weitere in Haarpflegemitteln übliche Bestandteile wie Parfums, Weichmacher, weitere natürliche und synthetische Harze, etc. mitverwendet werden.

Die folgenden Beispiele dienen der Illustration der Erfindung.

### Beispiel 1

| | |
|---|---|
| Aminopropyldimethylpolysiloxan/Polyethyloxazolin-Pfropfcopolymerisat | 30 (Gew.-%) |
| Isohexadecan | 40 |
| Ethanol | 30 |
| Parfum | q.s. |

Diese erfindungsgemäße Zusammensetzung wurde in einem Vergleichsversuch auf 10 Haarsträhnen mit gespaltenen Haarspitzen aufgebracht. Dies geschah in der Weise, daß die Lösung mit Daumen und Zeigefinger vom Haarende zur Haarspitze hin appliziert wurde. Nach fünfminütiger Einwirkungszeit wurden die Haarsträhnen gewaschen und manuell sowie visuell unter dem Stereolichtmikroskop begutachtet.

Parallel dazu wurden 10 Haarsträhnen in gleicher Weise mit einem dem Stand der Technik entsprechenden Präparat folgender Zusammensetzung behandelt:

| | |
|---|---|
| Nichtflüchtiges Dimethylpolysiloxan (Viskosität: 50.000 cSt bei 25°C) | 20 Gew.-% |
| Flüchtiges Dimethylpolysiloxan (Viskosität: 5 cSt bei 25°C) | 80 Gew.-% |

Bei der Auswertung zeigte sich, daß die mit den erfindungsgemäßen Produkten behandelten Haarspitzen vollständig zu einem einheitlichen Strang verbunden und keine Produktrückstände im Haar zurückgeblieben waren. Die mit dem bekannten Präparat behandelten Haarsträhnen zeigten eine ungleichmäßige Verklebung der Spitzen; das Präparat war darüber hinaus nicht vollständig aus dem Haar auswaschbar.

In den folgenden Beispielen 2 und 3 werden Zusammensetzungen beschrieben, deren Eigenschaften dem in Beispiel 1 wiedergegebenen Prodükt weitgehend entsprechen.

### Beispiel 2

| | |
|---|---|
| Aminopropyldimethylpolysiloxan/Polyethyloxazolin-Propfcopolymerisat | 25 (Gew.-%) |
| Cyclomethicone (Dow Corning ^{R} 245 Fluid) | 30 |
| Ethanol | 45 |
| Parfum | q.s. |

### Beispiel 3

| | |
|---|---|
| Aminopropyldimethylpolysiloxcan/Polyethyloxazolin-Pfropfcopolymerisat | 30 (Gew.-%) |
| Dimethicone (Dow Corning ^{R} 200 Fluid) | 40 |
| Ethanol | 30 |
| Parfum | q.s. |

## Patentansprüche

1. Mittel zur Haarbehandlung, enthaltend eine Lösung mindestens eines quaternierten Aminoalkyldimethylpolysiloxan/Polyethyloxazolin-Copolymerisats der Formel 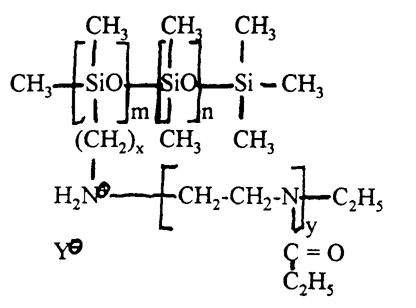 wobei m und n jeweils ganze Zahlen von 20 bis 10000, x eine Zahl zwischen 1 und 5 und y eine Zahl von 5 bis 500 bedeuten, und Y^{⊖} ein Anion darstellt, in mindestens einem organischen Lösungsmittel.

2. Mittel nach Anspruch 1, dadurch gekennzeichnet, daß es als Lösungsmittel Ethanol, n-Propanol und/oder Isopropylalkohol enthält.

3. Mittel nach Anspruch 1 oder 2, dadurch gekennzeichnet, daß es zusätzlich ein flüchtiges Silikon enthält.

4. Mittel nach einem oder mehreren der Ansprüche der 1 bis 5, dadurch gekennzeichnet, daß es zusätzlich einen Kohlenwasserstoff mit einem Siedepunkt zwischen 35°C und 280°C enthält.

5. Verwendung eines quaternierten Aminoalkyldimethylpolysiloxan/Polyethyloxazolin-Copolymerisats der Formel 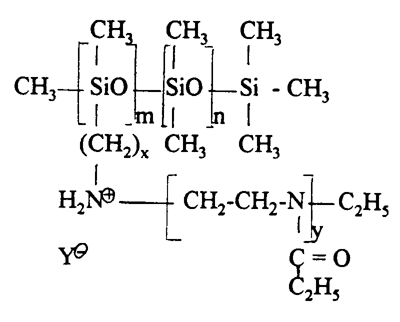 wobei m und n jeweils ganze Zahlen von 20 bis 10000, x eine Zahl zwischen 1 und 5 und y eine Zahl von 5 bis 500 bedeuten, und Y° ein Anion darstellt, in Präparaten zur Behandlung von gespaltenen Haarspitzen.

## Claims

1. Hair treatment composition comprising a solution of at least one quaternized aminoalkyl dimethyl polysiloxane/polyethyl oxazoline copolymer of the formula 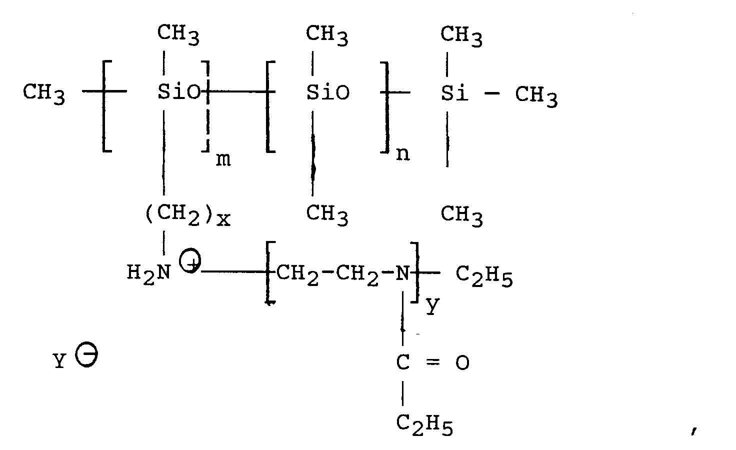 wherein m and n each denote integers from 20 to 10,000, x is a number between 1 and 5, and y is a number from 5 to 500, Y^{⊖} being an anion,
in at least one organic solvent.

2. Composition according to claim 1, characterized in that it comprises ethanol, n-propanol and (or) isopropyl alcohol as solvent.

3. Composition according to claims 1 or 2, characterized in that it additionally comprises a volatile silicone.

4. Composition according to one or more of the claims 1 to 5, characterized in that it additionally comprises a hydrocarbon having a boiling point between 35°C and 280°C.

5. Use of a quaternized aminoalkyl dimethyl polysiloxane/polyethyl oxazoline copolymer of the formula 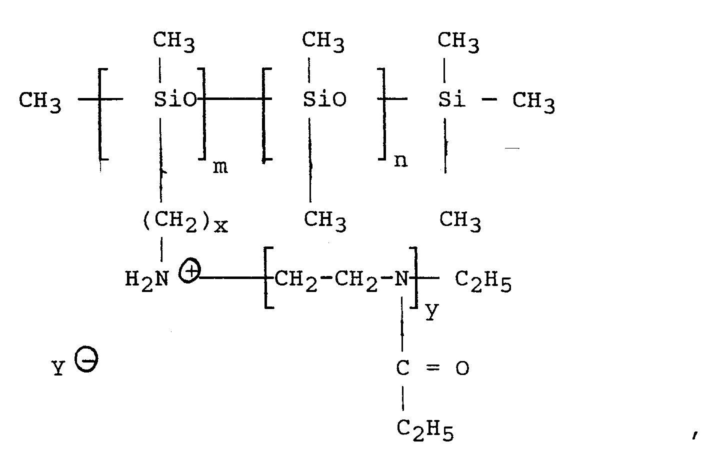 wherein m and n each stand for integers between 20 and 10,000, x is a number between 1 and 5, and y is a number from 5 to 500, Y^{⊖} being an anion, for compositions for the treatment of split hair ends.

## Revendications

1. Produit de traitement des cheveux, contenant une solution d'au moins un copolymère aminoalkyldiméthylpolysiloxane/polyéthyloxazoline quaternaire de formule 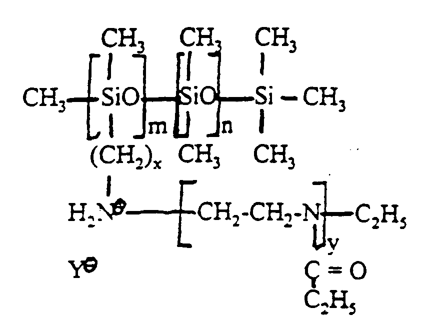 dans laquelle m et n représentent chacun un nombre entier allant de 20 à 10 000, x représente un nombre compris entre 1 et 5, et y représente un nombre allant de 5 à 500, et Y⁻ représente un anion, dans au moins un solvant organique.

2. Produit selon la revendication 1, caractérisé en ce qu'il contient comme solvant de l'éthanol, du n-propanol et/ou de l'alcool isopropylique.

3. Produit selon la revendication 1 ou 2, caractérisé en ce qu'il contient en outre une silicone volatile.

4. Produit selon une ou plusieurs des revendications 1 à 5, caractérisé en ce qu'il contient en outre un hydrocarbure ayant un point d'ébullition compris entre 35°C et 280°C.

5. Utilisation d'un copolymère aminoalkyldiméthylpolysiloxane/polyéthyloxazoline quaternaire de formule 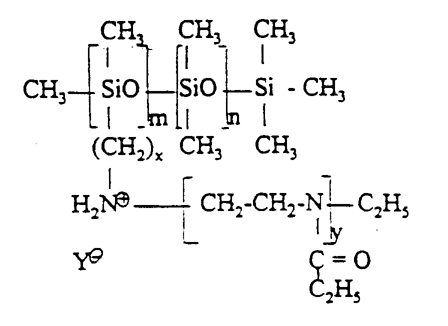 dans laquelle m et n représentent chacun un nombre entier allant de 20 à 10 000, x représente un nombre compris entre 1 et 5, et y représente un nombre allant de 5 à 500, et Y⁻ représente un anion, dans des compositions pour le traitement de pointes de cheveux fourchues.
